# EUROPEAN PATENT APPLICATION

(11) **EP 2 145 900 A1**
(43) Date of publication of application: **20.01.2010**
(21) Application number: 08012731.9
(22) Date of filing: 15.07.2008
(51) Int. Cl.: C07K 16/28, A61P 7/02

(54) **Orai1 (CRACM1) is the platelet SOC channel and essential for pathological thrombus formation**

(71) Applicant: CSL Behring GmbH, 35041 Marburg (DE); Julius-Maximilians-Universität Würzburg, 97070 Würzburg (DE)
(72) Inventor: Braun, Attila, 97070 Würzburg (DE); Nieswandt, Bernhard, 97246 Eibelstadt (DE); Varga-Szabó, Dávid, 42119 Wuppertal (DE)

(57) **Abstract**

The present invention relates to a pharmaceutical composition comprising an inhibitor of Orai1 or an inhibitor of an activator of Orai1 and optionally a pharmaceutically active carrier, excipient or diluent, for use in treating or preventing a disorder related to venous or arterial thrombus formation.

## Description

The present invention relates to a pharmaceutical composition comprising an inhibitor of Orai1 (also designated as CRACM1) or an inhibitor of an activator of Orai1 and optionally a pharmaceutically active carrier, excipient or diluent.

In this specification, a number of documents are cited. The disclosure content of these documents including manufacturer's manuals is herewith incorporated by reference in its entirety.

At sites of vascular injury the subendothelial extracellular matrix (ECM) is exposed to the flowing blood and triggers sudden platelet activation and the formation of a fibrin containing thrombus. This process is essential to prevent excessive posttraumatic blood loss but if it occurs at sites of atherosclerotic plaque rupture it can also lead to vessel occlusion and the development of myocardial infarction or ischemic stroke, which are among the leading causes of mortality and severe disability in industrialized countries (Ruggeri (2002); Nieswandt (2003)). Therefore, the inhibition of platelet activation has become an important strategy to prevent or treat such acute ischemic events (Bhatt (2003)). Platelet activation can occur through different signaling pathways which culminate in the activation of phospholipase (PL) C isoforms and production of diacylglycerol (DAG) and inositol 1,4,5-triphosphate (IP₃). IP₃ binds its receptors on the membrane of the intracellular Ca²⁺ stores and mediates Ca²⁺ release into the cytosol. In platelets, the dense tubular system, referred to as sarcoplasmic reticulum (SR) is thought to be the major Ca²⁺ store. The resulting decline in Ca²⁺ store content in turn triggers a sustained influx of extracellular Ca²⁺ by a mechanism known as store-operated Ca²⁺ entry (SOCE) (Berridge (2003); Feske (2007)). Although SOCE has pharmacologically been well defined for more than a decade, not much was known about the underlying molecular machinery until recently, when stromal interaction molecule 1 (STIM1) was identified as a sarco/endoplasmic (SR/ER)-store calcium sensor that controls SOCE in T cells and mast cells (Baba (2008); Liou; Oh-Hora (2008); Zhang (2005)). Shortly after that, we have shown that STIM1-mediated SOCE is critical for platelet function (Grosse (2007)), mainly by contributing to stable thrombus formation under flow conditions (Varga-Szabo (2008)). However, the identity of the store-operated Ca²⁺ (SOC) channel in platelets has remained elusive. Members of the transient receptor potential channel (TRPC) family, most notably TRPC1, have been proposed to contribute to SOCE in platelets (Rosado (2002); Sage (2002); Lopez (2006)) but the overall significance of this contribution has remained unclear because the expression rates of TRPCs in platelets are very low and mice lacking TRPC1 have no detectable defect in SOCE (Varga-Szabo (2008)).

Very recently the transmembrane protein Orai1 (also called CRACM1) has been identified as an essential component of SOCE in human T cells and mast cells (Feske (2006); Vig (2006); Vig (2008); Prakriya (2006); Yeromin (2006)). Orai1 was very recently shown to be expressed in human platelets (Tolhurst et al., Platelets, Volume 19, Issue 4, June 2008, pages 308 - 313). Whereas the authors speculate that STIM1:Orai1 acts as a primary pathway for agonist-evoked Ca²⁺ influx in the platelet and megakaryocyte, i.e. as key signal for platelet activation, there is, so far, no indication or evidence that Orai1 could be involved in the activation of platelet-mediated ischemic events. The authors disclose also no information about potentially unwanted or any additional, medically desired effects of reducing the function of Orai1, which would correspond to a therapeutic intervention at this receptor. Furthermore, based on the speculation of Tolhurst et al. on the key role of Orai1 for platelet activation, the skilled person would additionally predict that Orai1 is an unsuitable target for medical interventions, because Orai1 antagonists would at least inevitably result in serious hemostasis defects. Tolhurst et al. speculate even about lethal consequences of modulating Orai1 activity, citing a reference disclosing an increased embryonic lethality of transgenic mice, with elevated STIM1 activity (Grosse et al., J. Clin. Invest., Volume 117, Number 11, pages 3540-3550).

Despite the fact that thrombus formation leads to some of the most frequently occurring diseases in humans und despite extensive basic and clinical research that has been carried out in the field of thrombosis over decades, medicaments that have been registered and are presently available for patients are unsatisfactory for a variety of reasons. One problem common to all anti-coagulants presently used in clinics is their association with an increased risk of serious bleeding. These include heparins, cumarins, direct thrombin inhibitors such as hirudin, as well as aspirin, P2Y₁₂ inhibitors such as clopidogrel and GPIIb/IIIa inhibitors such as abciximab (ReoPro). On the other hand, many anticoagulants / antiplatelet agents have additional undesired effects such as the induction of thrombocytopenia. This is best described for heparin (heparin-induced thrombocytopenia, HIT) (Hassan, Y. et al., 2007, J. Clin. Pharm. Ther. 32:535-544) or GPIIb/IIIa blockers (abciximab, ReoPro) (Höchtl, T., 2007, J. Thromb. Thrombolysis. 24:59-64.). For other prominent inhibitors such as aspirin or the P2Y₁₂ inhibitor clopidogrel, many patients have been described as low or non-responders (Papthanasiou et al., 2007, Hellenic J. Cardiol. 48:352-363).

The technical problem underlying the present invention was to identify alternative and/or improved means and methods for successfully targeting diseases based on thrombus formation that form the basis or may allow the development of more satisfactory medicaments for the treatment and/or prevention of the mentioned diseases.

The solution to this technical problem is achieved by providing the embodiments characterized in the claims.

Accordingly, the present invention relates to a pharmaceutical composition comprising an inhibitor of Orai1 or an inhibitor of an activator of Orai1 and optionally a pharmaceutically active carrier, excipient or diluent.

The term "pharmaceutical composition" as employed herein comprises at least one such as at least two, e.g. at least three, in further embodiments at least four such as at last five of the mentioned inhibitors. The invention also envisages mixtures of inhibitors of Orai1 and inhibitors of an activator of Orai1.

The composition may be in solid, liquid or gaseous form and may be, inter alia, in a form of (a) powder(s), (a) tablet(s), (a) solution(s) or (an) aerosol(s).

It is preferred that said pharmaceutical composition comprises a pharmaceutically acceptable carrier, excipient and/or diluent. Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. It is particularly preferred that said administration is carried out by injection and/or delivery, e.g., to a site in the bloodstream such as a brain or coronary artery or directly into the respective tissue. The compositions of the invention may also be administered directly to the target site, e.g., by biolistic delivery to an external or internal target site, like the brain or the heart. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Proteinaceous pharmaceutically active matter may be present in amounts between 1 ng and 10 mg/kg body weight per dose; however, doses below or above this exemplary range are envisioned, especially considering the aforementioned factors. If the regimen is a continuous infusion, it should also be in the range of 0.01 µg to 10 mg units per kilogram of body weight per minute. The continuous infusion regimen may be completed with a loading dose in the dose range of 1 ng and 10 mg/kg body weight.

Progress can be monitored by periodic assessment. The compositions of the invention may be administered locally or systemically. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. It is particularly preferred that said pharmaceutical composition comprises further agents known in the art to antagonize thrombus formation or to reduce thrombus size. Since the pharmaceutical preparation of the present invention relies on the above mentioned inhibitors, it is preferred that those mentioned further agents are only used as a supplement, i.e. at a reduced dose as compared to the recommended dose when used as the only drug, so as to e.g. reduce side effects conferred by the further agents. Conventional excipients include binding agents, fillers, lubricants and wetting agents.

The term "inhibitor of Orai1" refers to an inhibitor that reduces the biological function of Orai1 to at least 50%, preferably to at least 75%, more preferred to at least 90% and even more preferred to at least 95% such as at least 98% or even at least 99%. Biological function denotes in particular any known biological function of Orai1 or any combination thereof including functions elucidated in accordance with the present invention. Examples of said biological function are the binding of Orai1 to STIM1 or STIM2 (Oh-Hora (2008); Zhang (2005); Putney JW Jr,, 2007, Cell Calcium 42(2):103-110) or other regulators, its function to act as a store-operated Ca²⁺ (SOC) channel, its mediation of SOCE, and in optional conjunction therewith its requirement for the stabilization of platelet-rich thrombi at sites of arterial injury under conditions where the process is mainly driven by GPIb-GPVI-ITAM dependent mechanisms, further optionally in conjunction with mediation of SOCE, the impairment of integrin activation and degranulation, its role in homeostasis of platelets, the contribution to the formation of thrombus formation such as three dimensional thrombus formation, in particular pathologic occlusive thrombus formation (platelet-rich thrombi), and the contribution to platelet activation. All these functions can be tested for by the skilled person either on the basis of common general knowledge or on the basis of the teachings of this specification, optionally in conjunction with the teachings of the documents cited therein.

The term "inhibitor of an activator of Orai1" refers to inhibitors that do not directly interact with Orai1 but with molecules that directly or indirectly activate one or more of the biological functions of Orai1 and preferably one or more of those functions referred to above or elsewhere in this specification. Non-limiting examples of molecules that directly or indirectly activate one or more of the biological functions of Orai1 include STIM1 and STIM2. The inhibition values referred to above for inhibitors of Orai1 mutatis mutandis apply to inhibitors of an activator of Orai1.

In accordance with the present invention, we show that Orai1 is strongly expressed in human and mouse platelets. Analysis of Orai1^{-/-} mice revealed an essential role of the channel in platelet SOCE and thrombus formation in vitro and in vivo. However, anti-coagulants, including those anti-coagulants presently used in clinics, have the draw-back of being associated with an increased risk of serious bleeding. Thus, the function of Orai1 in platelet SOCE and thrombus formation would lead the skilled person to expect serious hemostasis defects when using Orai1 inhibitors. However, the present inventors show that a lack of Orai1 biological function can prevent unwanted thrombus formation in bloodstream such as in a brain or coronary artery without being associated with increased occurrence of bleeding. Thus, the present invention overcomes a major obstacle in current stroke and myocardial infarction treatment.

In accordance with the present invention, it is shown that Orai1 is the principal SOC channel in platelets and that its absence leads to a similarly severe defect in SOCE as the absence of STIM1 (Varga-Szabo (2008)). This finding is unanticipated given previous reports that suggested an important role of channels of the TRPC family, most notably TRPC1 in this process (Rosado (2002), Sage (2002), Lopez (2006)). Our data do not exclude the possibility that TRPC1 contributes to SOCE in platelets. STIM1 has been shown to interact not only with Orai1 but also with members of the TRPC family, including TRPC1 (Huang (2006)) and to activate them directly and indirectly by the formation of heteromultimers, indicating that TRPC1 could be part of a channel complex that is regulated by STIM1 (Yuan (2007)). However, irrespective of the exact mechanism how TRPC1 may be involved in SOCE in platelets this contribution is not essential as revealed by the recent analysis of TRPC1^{-/-} mice, which showed no detectable defect in platelet SOCE and cellular activation in vitro and in vivo (Varga-Szabo (2008)).

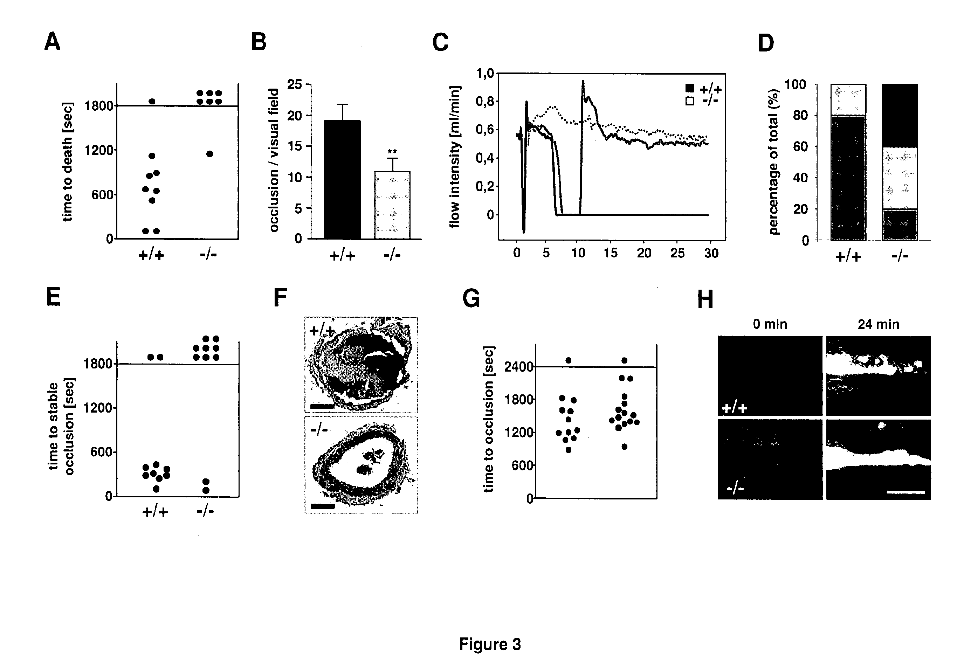

Lack of Orai1 resulted in strongly reduced SOCE in response to the thapsigargin (TG) an inhibitor of the sarcoplasmic/endoplasmic reticulum Ca²⁺ ATPase (SERCA) and all major physiological agonists but in contrast to STIM1-deficiency it had no effect on the filling state of the Ca²⁺ store. Similar observations have previously been made in Orai1^{-/-} and Stim1^{-/-} mast cells (Baba (2008); Vig (2008)). This shows that functional SOCE is not a prerequisite of proper store refill and indicates that STIM1 presumably plays a direct, yet unidentified, role in this process. Although the difference in agonist-induced Ca²⁺ store release between Orai1^{-/-} and Stim1^{-/-} platelets is rather small, it may still be physiologically relevant. This became most evident when FeCl₃-induced thrombus formation was assessed in mesenteric vessels (Fig. 3). Orai1^{-/-} chimeras were able to form stable thrombi in this model, whereas no occlusive thrombus formation is seen in Stim1^{-/-} chimeras under the same experimental conditions (Varga-Szabo (2008)). This indicates that the relatively small increase in [Ca²⁺]ᵢ caused by store release in platelets may be sufficient to drive thrombus formation independently of SOCE under certain conditions. As platelets have to respond to vascular injury very rapidly, it appears plausible that the first adhesion and activation is regulated mainly by Ca²⁺ from the stores and very fast Ca²⁺ channels such as the ATP-gated P2X1 channel, which has been shown to be critical for proper platelet recruitment and activation at very high shear rates (Hechler (2003)). However, SOCE appears to be of pivotal importance for thrombus stabilization on collagen/vWF substrates under conditions of high shear which is predominantly mediated by the GPIb-GPVI-ITAM axis (Ruggeri (2002), Nieswandt (2003)). This was also confirmed by the virtually complete protection of Orai1^{-/-} chimeras from tMCAO-induced neuronal damage which was comparable to the protection seen in Stim1^{-/-} chimeras (Varga-Szabo (2008)). The development of large brain infarcts in this model is known to be highly dependent on functional GPIb and to a somewhat lesser extent also GPVI (Kleinschnitz (2007)), indicating that STIM1/Orai1-dependent SOCE may indeed occur predominantly downstream of these receptors during intracerebral thrombus formation following transient ischemia. Importantly, this marked protection was not associated with increased occurrence of intracranial bleeding which is still the major obstacle in current stroke treatment (Bhatt (2003)). In line with this, we observed only a minor increase in tail bleeding times in Orai1^{-/-} chimeras suggesting that Orai1, like STIM1, may be of greater relative significance for arterial thrombus formation than for primary hemostasis. Taken together, our results establish Orai1 as the long-sought platelet SOC channel that is of central importance for platelet activation during arterial thrombosis and ischemic brain infarction. Since Orai1 is expressed in the plasma membrane and because its inhibition overcomes a major obstacle in current stroke and myocardial infarction treatment, namely an increased risk of serious bleeding, it may be an even more preferred target for pharmacological inhibition as compared to STIM1 to prevent or treat ischemic cardio- and cerebrovascular diseases.

Further, the invention relates to an inhibitor of Orai1 or an inhibitor of an activator of Orai1 for use in treating or preventing a disorder related to venous or arterial thrombus formation. Alternatively, the mentioned inhibitor may be used as a lead compound for the development of a drug for treating or preventing a disorder related to venous or arterial thrombus formation. Those lead compounds will also allow for the development of novel, highly effective, yet safe antithrombotics. In the development of those drugs, the following developments are considered: (i) modified site of action, spectrum of activity, organ specificity, and/or (ii) improved potency, and/or (iii) decreased toxicity (improved therapeutic index), and/or (iv) decreased side effects, and/or (v) modified onset of therapeutic action, duration of effect, and/or (vi) modified pharmacokinetic parameters (resorption, distribution, metabolism and excretion), and/or (vii) modified physico-chemical parameters (solubility, hygroscopicity, color, taste, odor, stability, state), and/or (viii) improved general specificity, organ/tissue specificity, and/or (ix) optimized application form and route by (i) esterification of carboxyl groups, or (ii) esterification of hydroxyl groups with carboxylic acids, or (iii) esterification of hydroxyl groups to, e.g. phosphates, pyrophosphates or sulfates or hemi-succinates, or (iv) formation of pharmaceutically acceptable salts, or (v) formation of pharmaceutically acceptable complexes, or (vi) synthesis of pharmacologically active polymers, or (vii) introduction of hydrophilic moieties, or (viii) introduction/exchange of substituents on aromates or side chains, change of substituent pattern, or (ix) modification by introduction of isosteric or bioisosteric moieties, or (x) synthesis of homologous compounds, or (xi) introduction of branched side chains, or (xii) conversion of alkyl substituents to cyclic analogues, or (xiii) derivatisation of hydroxyl group to ketales, acetales, or (xiv) N-acetylation to amides, phenylcarbamates, or (xv) synthesis of Mannich bases, imines, or (xvi) transformation of ketones or aldehydes to Schiffs bases, oximes, acetales, ketales, enolesters, oxazolidines, thiazolidines or combinations thereof.

The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000).

Further, the present invention relates to a method of treating or preventing a disorder related to venous or arterial thrombus formation comprising administering a pharmaceutically effective amount of an inhibitor of Orai1 or of an inhibitor of an activator of Orai1 to a subject in need thereof.

In a preferred embodiment of the pharmaceutical composition or the inhibitor of the invention, the inhibitor is an antibody or fragments thereof, an aptamer, a siRNA, a shRNA, a miRNA, a ribozyme, an antisense nucleic acid molecule, modified versions of these inhibitors or a small molecule.

The antibody in accordance with the present invention can be, for example, polyclonal or monoclonal. The term "antibody" also comprises derivatives or fragments thereof which still retain the binding specificity. Techniques for the production of antibodies are well known in the art and described, e.g. in Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999.

The antibody also includes embodiments such as chimeric (human constant domain, non-human variable domain), single chain and humanized (human antibody with the exception of non-human CDRs) antibodies, as well as antibody fragments, like, inter alia, Fab, or Fab' fragments. Antibody fragments or derivatives further comprise Fd, F(ab')₂, Fv or scFv fragments; see, for example, Harlow and Lane (1988) and (1999), loc. cit.. Various procedures are known in the art and may be used for the production of such antibodies and/or fragments. Thus, the (antibody) derivatives can be produced by peptidomimetics. Further, techniques described for the production of single chain antibodies (see, inter alia, US Patent 4,946,778) can be adapted to produce single chain antibodies specific for polypeptide(s) and fusion proteins of this invention. Also, transgenic animals or plants (see, e.g., US patent 6,080,560) may be used to express (humanized) antibodies specific for the target of this invention. Most preferably, the antibody is a monoclonal antibody, such as a human or humanized antibody. For the preparation of monoclonal antibodies, any technique which provides antibodies produced by continuous cell line cultures can be used. Examples for such techniques include the hybridoma technique (Köhler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BIAcore system can be used to increase the efficiency of phage antibodies which bind to an epitope of Orai1 of an activator of Orai1 (Schier, Human Antibodies Hybridomas 7 (1996), 97-105; Malmborg, J. Immunol. Methods 183 (1995), 7-13). It is also envisaged in the context of this invention that the term "antibody" comprises antibody constructs which may be expressed in cells, e.g. antibody constructs which may be transfected and/or transduced via, inter alia, viruses or plasmid vectors.

Aptamers are oligonucleic acid or peptide molecules that bind a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications (Osborne et. al. (1997), Current Opinion in Chemical Biology, 1:5-9; Stull & Szoka (1995), Pharmaceutical Research, 12, 4:465-483). More specifically, aptamers can be classified as DNA or RNA aptamers or peptide aptamers. Whereas the former normally consist of (usually short) strands of oligonucleotides, the latter preferably consist of a short variable peptide domain, attached at both ends to a protein scaffold.

Nucleic acid aptamers are nucleic acid species that, as a rule, have been engineered through repeated rounds of in vitro selection or equivalently, SELEX (systematic evolution of ligands by exponential enrichment) to bind to various molecular targets such as small molecules, proteins, nucleic acids, and even cells, tissues and organisms.

Peptide aptamers usually are peptides or proteins that are designed to interfere with other protein interactions inside cells. They consist of a variable peptide loop attached at both ends to a protein scaffold. This double structural constraint greatly increases the binding affinity of the peptide aptamer to levels comparable to an antibody's (nanomolar range). The variable loop length is typically comprised of 10 to 20 amino acids, and the scaffold may be any protein which have good solubility properties. Currently, the bacterial protein Thioredoxin-A is the most used scaffold protein, the variable loop being inserted within the reducing active site, which is a -Cys-Gly-Pro-Cys- loop in the wild protein, the two cysteins lateral chains being able to form a disulfide bridge. Peptide aptamer selection can be made using different systems, but the most used is currently the yeast two-hybrid system.

Aptamers offer the utility for biotechnological and therapeutic applications as they offer molecular recognition properties that rival those of the commonly used biomolecules, in particular antibodies. In addition to their discriminate recognition, aptamers offer advantages over antibodies as they can be engineered completely in a test tube, are readily produced by chemical synthesis, possess desirable storage properties, and elicit little or no immunogenicity in therapeutic applications.

Non-modified aptamers are cleared rapidly from the bloodstream, with a half-life of minutes to hours, mainly due to nuclease degradation and clearance from the body by the kidneys, a result of the aptamer's inherently low molecular weight. Unmodified aptamer applications currently focus on treating transient conditions such as blood clotting, or treating organs such as the eye where local delivery is possible. This rapid clearance can be an advantage in applications such as in vivo diagnostic imaging. Several modifications, such as 2'-fluorine-substituted pyrimidines, polyethylene glycol (PEG) linkage, fusion to albumin, albumin-like proteins or other half life extending proteins like Fc parts of antibodies are available to scientists with which the half-life of aptamers easily can be increased to the day or even week time scale.

The term "peptide" as used herein describes a group of molecules consisting of up to 30 amino acids, whereas "proteins" consist of more than 30 amino acids. Peptides and proteins may further form dimers, trimers and higher oligomers, i.e. consisting of more than one molecule which may be identical or non-identical. The corresponding higher order structures are, consequently, termed homo- or heterodimers, homo- or heterotrimers etc. The terms "peptide" and "protein" (wherein "protein" is interchangeably used with "polypeptide") also refer to naturally modified peptides/proteins wherein the modification is effected e.g. by glycosylation, acetylation, phosphorylation and the like. Such modifications are well-known in the art.

For therapeutic uses, the RNA inactivation by antisense molecules or by ribozymes is implementable. Both classes of compounds can be synthesized chemically or produced in conjunction with a promoter by biological expression in vitro or even in vivo.

Small interfering RNA (siRNA), sometimes known as short interfering RNA or silencing RNA, are a class of 18 to 30, preferably 19 to 25, most preferred 21 to 23 or 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome.

Natural siRNAs have a well defined structure: a short double-strand of RNA (dsRNA) with 2-nt 3' overhangs on either end. Each strand has a 5' phosphate group and a 3' hydroxyl (-OH) group. This structure is the result of processing by dicer, an enzyme that converts either long dsRNAs or small hairpin RNAs into siRNAs. SiRNAs can also be exogenously (artificially) introduced into cells to bring about the specific knockdown of a gene of interest. Essentially any gene of which the sequence is known can thus be targeted based on sequence complementarity with an appropriately tailored siRNA.

The double-stranded RNA molecule or a metabolic processing product thereof is capable of mediating target-specific nucleic acid modifications, particularly RNA interference and/or DNA methylation. Preferably at least one RNA strand has a 5'-and/or 3'-overhang. Preferably, one end of the double-strand has a 3'-overhang from 1-5 nucleotides, more preferably from 1-3 nucleotides and most preferably 2 nucleotides. The other end may be blunt-ended or has up to 6 nucleotides 3'-overhang. In general, any RNA molecule suitable to act as siRNA is envisioned in the present invention.

The most efficient silencing was so far obtained with siRNA duplexes composed of 21-nt sense and 21-nt antisense strands, paired in a manner to have a 2-nt 3'-overhang. The sequence of the 2-nt 3' overhang makes a small contribution to the specificity of target recognition restricted to the unpaired nucleotide adjacent to the first base pair (Elbashir et al. 2001). 2'-deoxynucleotides in the 3' overhangs are as efficient as ribonucleotides, but are often cheaper to synthesize and probably more nuclease resistant.

A short hairpin RNA (shRNA) is a sequence of RNA that makes a tight hairpin turn that can be used to silence gene expression via RNA interference. shRNA uses a vector introduced into cells and utilizes the U6 promoter to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs which match the siRNA that is bound to it.

Si/shRNAs to be used in the present invention are preferably chemically synthesized using appropriately protected ribonucleoside phosphoramidites and a conventional DNA/RNA synthesizer. Suppliers of RNA synthesis reagents are Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA), and Cruachem (Glasgow, UK). Most conveniently, siRNAs or shRNAs are obtained from commercial RNA oligo synthesis suppliers, which sell RNA-synthesis products of different quality and costs. In general, the RNAs applicable in the present invention are conventionally synthesized and are readily provided in a quality suitable for RNAi.

Further molecules effecting RNAi include, for example, microRNAs (miRNA). Said RNA species are single-stranded RNA molecules which as endogenous RNA molecules regulate gene expression. Upon binding to a complementary mRNA transcript triggers the degradation of said mRNA transcript through a process similar to RNA interference. Accordingly, miRNAs may be employed to regulate the expression of Orai1.

A ribozyme (from ribonucleic acid enzyme, also called RNA enzyme or catalytic RNA) is an RNA molecule that catalyzes a chemical reaction. Many natural ribozymes catalyze either their own cleavage or the cleavage of other RNAs, but they have also been found to catalyze the aminotransferase activity of the ribosome.

Examples of well-characterized small self-cleaving RNAs are the hammerhead, hairpin, hepatitis delta virus, and in vitro-selected lead-dependent ribozymes. The organization of these small catalysts is contrasted to that of larger ribozymes, such as the group I intron.

The principle of catalytic self-cleavage has become well established in the last 10 years. The hammerhead ribozymes are characterized best among the RNA molecules with ribozyme activity. Since it was shown that hammerhead structures can be integrated into heterologous RNA sequences and that ribozyme activity can thereby be transferred to these molecules, it appears that catalytic antisense sequences for almost any target sequence can be created, provided the target sequence contains a potential matching cleavage site.

The basic principle of constructing hammerhead ribozymes is as follows: An interesting region of the RNA, which contains the GUC (or CUC) triplet, is selected. Two oligonucleotide strands, each usually with 6 to 8 nucleotides, are taken and the catalytic hammerhead sequence is inserted between them. Molecules of this type were synthesized for numerous target sequences. They showed catalytic activity in vitro and in some cases also in vivo. The best results are usually obtained with short ribozymes and target sequences.

A recent development, also useful in accordance with the present invention, is the combination of an aptamer recognizing a small compound with a hammerhead ribozyme. The conformational change induced in the aptamer upon binding the target molecule, is supposed to regulate the catalytic function of the ribozyme. The term "antisense nucleic acid molecule" is known in the art and refers to a nucleic acid which is complementary to a target nucleic acid. An antisense molecule according to the invention is capable of interacting with, more specifically hybridizing with the target nucleic acid. By formation of the hybrid, transcription of the target gene(s) and/or translation of the target mRNA is reduced or blocked. Standard methods relating to antisense technology have been described (see, e.g., Melani et al., Cancer Res. (1991) 51:2897-2901).

The term "modified versions of these inhibitors" refers to versions of the inhibitors that are modified to achieve i) modified spectrum of activity, organ specificity, and/or ii) improved potency, and/or iii) decreased toxicity (improved therapeutic index), and/or iv) decreased side effects, and/or v) modified onset of therapeutic action, duration of effect, and/or vi) modified pharmacokinetic parameters (resorption, distribution, metabolism and excretion), and/or vii) modified physico-chemical parameters (solubility, hygroscopicity, color, taste, odor, stability, state), and/or viii) improved general specificity, organ/tissue specificity, and/or ix) optimised application form and route by (a) esterification of carboxyl groups, or (b) esterification of hydroxyl groups with carboxylic acids, or (c) esterification of hydroxyl groups to, e.g. phosphates, pyrophosphates or sulfates or hemi-succinates, or (d) formation of pharmaceutically acceptable salts, or (e) formation of pharmaceutically acceptable complexes, or (f) synthesis of pharmacologically active polymers, or (g) introduction of hydrophilic moieties, or (h) introduction/exchange of substituents on aromates or side chains, change of substituent pattern, or (i) modification by introduction of isosteric or bioisosteric moieties, or (j) synthesis of homologous compounds, or (k) introduction of branched side chains, or (k) conversion of alkyl substituents to cyclic analogues, or (I) derivatisation of hydroxyl groups to ketales, acetales, or (m) N-acetylation to amides, phenylcarbamates, or (n) synthesis of Mannich bases, imines, or (o) transformation of ketones or aldehydes to Schiffs bases, oximes, acetales, ketales, enolesters, oxazolidines, thiazolidines; or combinations thereof.

The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000).

A small molecule may be, for example, an organic molecule. Organic molecules relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. Alternatively the compound may be an inorganic compound. Inorganic compounds are derived from mineral sources and include all compounds without carbon atoms (except carbon dioxide, carbon monoxide and carbonates). Preferably, the small molecule has a molecular weight of less than about 2000 amu, or less than about 1000 amu such as 500 amu, and even less than about 250 amu. The size of a small molecule can be determined by methods well-known in the art, e.g., mass spectrometry. The small molecules may be designed, for example, based on the crystal structure of STIM1 where sites presumably responsible for the biological activity, can be identified and verified in in vivo assays such as in vivo HTS assays.

All other inhibitors may also be identified and/or their function verified in HTS assays. High-throughput assays, independently of being biochemical, cellular or other assays, generally may be performed in wells of microtiter plates, wherein each plate may contain 96, 384 or 1536 wells. Handling of the plates, including incubation at temperatures other than ambient temperature, and bringing into contact of test compounds with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibits biological activity, said mixture of test compounds may be de-convoluted to identify the one or more test compounds in said mixture giving rise to said activity.

The determination of binding of potential inhibitors can be effected in, e.g., any binding assay, preferably biophysical binding assay, which may be used to identify binding test molecules prior to performing the functional/activity assay with theinhibitor. Suitable biophysical binding assays are known in the art and comprise fluorescence polarization (FP) assay, fluorescence resonance energy transfer (FRET) assay and surface plasmon resonance (SPR) assay.

In a further alternative method of identifying inhibitors, it is tested for inhibition of function in a cell transfected with said polynucleotide encoding the inhibitor if the inhibitor is of proteinaceous nature. This embodiment relates to a cellular screen. In a cellular screen inhibitors may be identified which exert their inhibitory activity by physically interacting with the target molecule, or alternatively (or additionally) by functionally interacting with said target molecule, i.e., by interfering with the pathway(s) present in the cells employed in the cellular assay.

The pharmaceutical composition of any one of claims 1 or 4 or 5 further comprising in the same or a separate container an antagonist of G-protein coupled receptors or signaling pathways, such as P2Y₁ inhibitors, P2Y₁₂ inhibitors, aspirin, inhibitors of PAR receptors.

The inhibitor of the invention, preferably in the pharmaceutical composition may have admixed thereto other anti-coagulants which are known in the art, described, for example, in WO2006/066878 which is specifically incorporated herein in its entirety.

The inhibitor of the invention may have admixed thereto or associated in a separate container an antagonist of G-protein coupled receptors or signalling pathways.

In a further preferred embodiment, the pharmaceutical composition or the inhibitor of the invention, the antagonist of G-protein coupled receptors or signaling pathways is an inhibitor of PAR receptors.

In another preferred embodiment of the inhibitor of the invention, the disorder related to venous or arterial thrombus formation is myocardial infarction, stroke, inflammation, complement activation, fibrinolysis, angiogenesis and/or diseases related to FXII-induced kinin formation such as hereditary angioedema, bacterial infection of the lung, trypanosome infection, hypotensitive shock, pancreatitis, chagas disease, thrombocytopenia or articular gout.

A myocardial infarction as used in the present invention relates to medical condition generally referred to as "heart attack" and is characterized by interrupted blood supply to the heart. The resulting ischemia (oxygen shortage) causes cellular damage and - depending on the length of ischemia - even tissue necrosis. Most commonly, myocardial infarct is due to the rupture of a vulnerable plaque that leads to a blockade of a vein or artery.

The term "stroke" is well-known in the art and sometimes also referred to as cerebrovascular accident (CVA). A stroke is a medical condition that is medically defined by reduced blood supply to the brain resulting in loss of brain function, inter alia due to ischemia. Said reduction in blood supply can be caused, for example, by thrombosis or embolism, or due to hemorrhage. Hence, strokes are generally classified into two major categories, ie., i) ischemic and ii) hemorrhagic strokes. Ischemia is due to an interruption in blood circulation and hemorrhage is due to a rupture of a blood vessel, both scenarios ultimately leading to a reduced blood supply of the brain. The prevalent form of stroke is the ischemic stroke accounting for about 80% of strokes. In an ischemic stroke, blood supply to part of the brain is decreased, leading to dysfunction and necrosis of the brain tissue in that area. There are mainly four causative reasons: thrombosis (obstruction of a blood vessel by a blood clot forming locally), embolism (idem due to a blood clot from elsewhere in the body), systemic hypoperfusion (general decrease in blood supply, e.g. in shock) and venous thrombosis.

Despite the importance of ischemic stroke as the third leading cause of death and disability in industrialized countries treatment options in acute stroke are limited (22). Numerous attempts to attenuate infarct progression in acute stroke patients by conventional platelet aggregation inhibitors or anti-coagulation failed due to an excess of intracerebral hemorrhages ( Toyoda K. et al., 2005, Neurology, Volume 65(7), page 1000-1004) In accordance with the present invention, it was found that Orai1^{-/-} chimeras are protected from neuronal damage following transient cerebral ischemia without displaying an increased risk of intracranial hemorrhage.

In a particularly preferred embodiment of the inhibitor of the invention, said stroke is therefore ischemic stroke.

### Figures

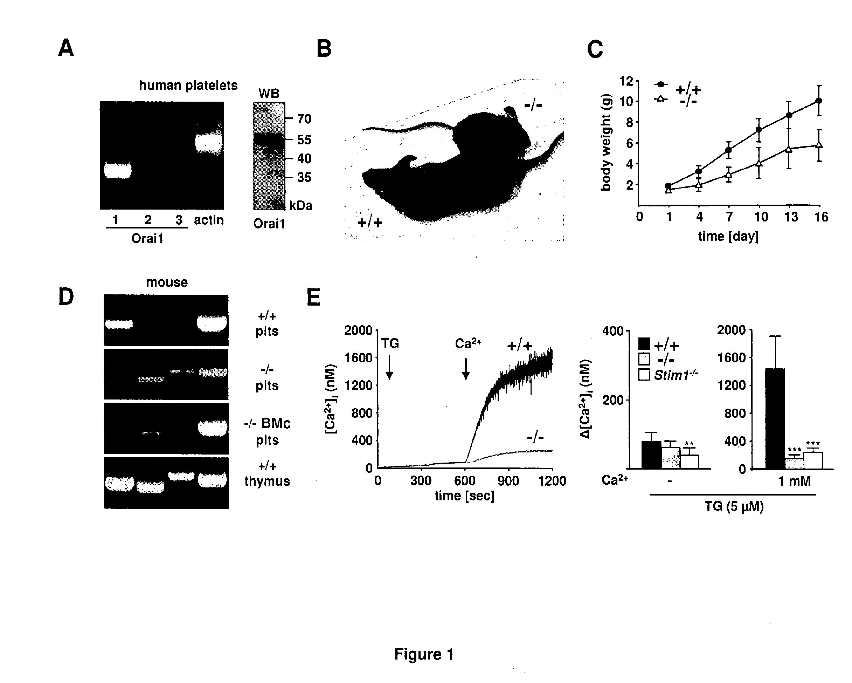

Figure 1. Orai1 is the platelet SOC channel. **(A)** RT-PCR and Western-blot analysis of human platelets. Orai1, 2 and 3 were assessed with the primer pairs described under materials and methods, and Western-blot was performed using an antibody from ProSci Inc. **(B)** Wild-type and Orai1^{-/-} littermates, 3 weeks old. **(C)** Body weights of wild-type (+/+) and Orai1^{-/-} (-/-) mice. **(D)** RT-PCR analyses of platelet and thymocyte mRNA from wild-type (+/+), original Orai1^{-/-} (-/-) and Orai1^{-/-} bone marrow chimera (-/- BMc) mice. Orai1, 2 and 3 specific forward and reverse primers were used (21), actin served as control. **(E)** Fura-2-loaded platelets were stimulated with 5 µM TG for 10 min followed by addition of 1 mM extracellular Ca²⁺, and monitoring of [Ca²⁺]ᵢ. Representative measurements (left) and maximal Δ[Ca ²⁺]ᵢ ± SD (n = 4 per group) before and after addition of 1 mM Ca²⁺ (right) are shown. The white bars represent Stim1^{-/-} platelets.

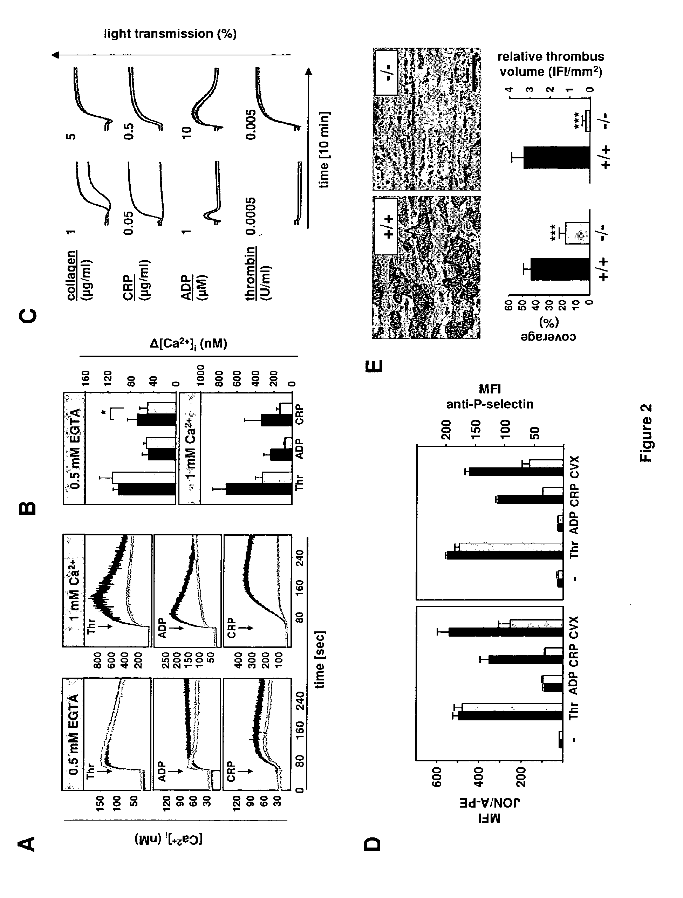

Figure 2. Defective agonist-induced Ca²⁺-response and aggregate formation under flow in Orai1^{-/-} platelets. Fura-2-loaded wild-type (black line) or Orai1^{-/-} (grey line) platelets were stimulated with thrombin (0.1 U/ml), ADP (10 µM) or CRP (10 µg/ml) in calcium-free medium or in the presence of extracellular Ca²⁺ (1 mM), and [Ca²⁺], was monitored. Representative measurements **(A)** and maximal Δ[Ca ²⁺]ᵢ ± SD (n = 4 per group) **(B)** are shown. **(C)** Impaired aggregation of Orai1^{-/-} platelets (grey lines) in response to collagen, but not ADP and thrombin. **(D)** Flow cytometric analysis of GPIIb-IIIa integrin activation (left panel) and degranulation-dependent P-selectin exposure (right panel) in response to thrombin (0.1 U/ml), ADP (10 µM), CRP (10 µg/ml) and CVX (1 µg/ml). Results are means ± SD of 6 mice per group. **(E)** Orai1^{-/-} platelets in whole blood fail to form stable thrombi when perfused over a collagen-coated (0.2 mg/ml) surface at a shear rate of 1.700 s⁻¹. Upper: representative phase contrast images. Lower: Mean surface coverage (left) and relative platelet deposition as measured by the integrated fluorescent intensity (IFI) per mm² (right) ± SD (n=4). Bar represents 30 µm.
Figure 3. Reduced thrombus stability of Orai1^{-/-} platelets in vivo. **(A-B)** Lethal pulmonary embolization after injection of collagen and epinephrine in anesthetized wild-type (+/+) and Orai1^{-/-} (-/-) mice. **(A)** Time to death through asphyxia. Each symbol represents one individual. **(B)** Occluded arteries in the harvested lungs per visual field. **(C-F)** Mechanical injury of the abdominal aorta of wild-type (+/+) and Orai1^{-/-} (-/-) mice was performed and blood flow was monitored with a Doppler flowmeter. Representative flow measurements **(C),** per cent distribution of irreversible occlusion (dark grey), unstable occlusion (light grey) and no occlusion (black) **(D),** time to final occlusion (each symbol represents one individual) **(E)** and representative cross-sections of the aorta 30 min after injury **(F)** are shown. Bars represent 100 µm. **(G-H)** FeCl₃ induced chemical injury of small mesenteric arteries from wild-type (+/+) and Orai1^{-/-} (-/-) chimeras. **(G)** Time to occlusion. Each dot represents one individual. (H) Representative fluorescent images before and 24 min after injury. Bar represents 50 µm.

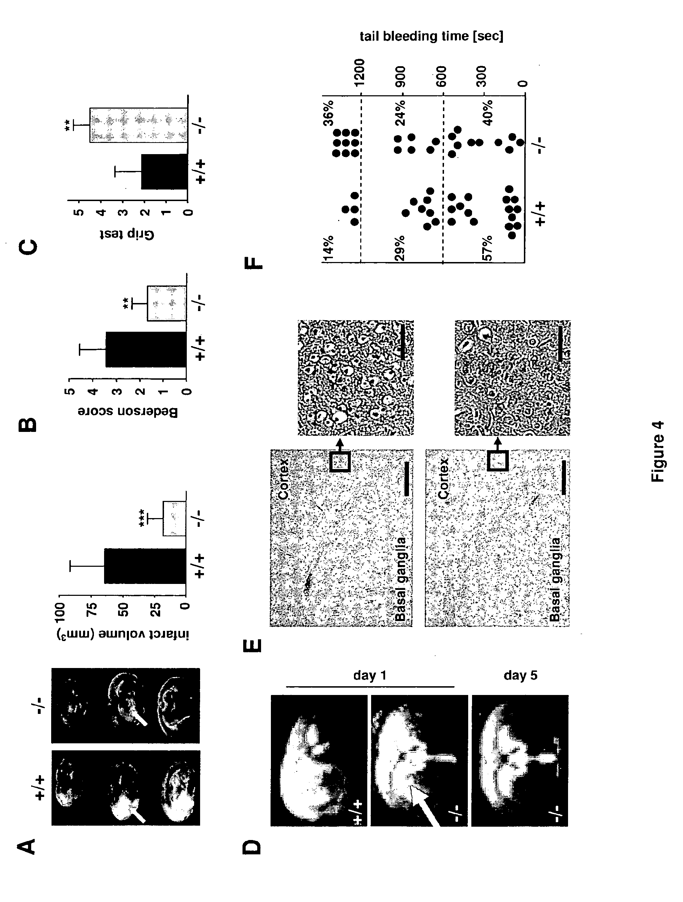

Figure 4. Orai1^{-/-} chimeras are protected from cerebral ischemia without displaying major bleeding. **(A)** Left panel: Representative images of three corresponding coronal sections from control and Orai1^{-/-} chimeras mice stained with TTC 24 hrs after tMCAO. Infarct areas marked with arrows. Right panel: Brain infarct volumes in control (n=7) and Orai1^{-/-} chimeras (n=7), *** p<0.0001. **(B, C)** Neurological Bederson score and grip test assessed at day 1 following tMACO of control (n=7) and Orai1^{-/-} chimeras (n=7), ** p<0.01. **(D)** The coronal T2-w MR brain image shows a large hyperintense ischemic lesion at day 1 after tMCAO in controls (left). Infarcts are smaller in Orai1^{-/-} chimeras (middle, white arrow), and T2-hyperintensity decreases by day 5 during infarct maturation (right). Importantly, hypointense areas indicating intracerebral hemorrhage were not seen in Orai1^{-/-} chimeras, demonstrating that Orai1 deficiency does not increase the risk of hemorrhagic transformation, even at advanced stages of infarct development. **(E)** Hematoxylin and eosin stained sections of corresponding territories in the ischemic hemispheres of control and Orai1^{-/-} chimeras. Infarcts are restricted to the basal ganglia in Orai1^{-/-} chimeras but consistently include the cortex in controls. Magnification x 100-fold. Bars represent 300 µm (left) and 37.5 µm (right). **(F)** Bleeding time is only mildly prolonged in Orai1^{-/-} chimeras after amputating the tail tip of anesthetized mice. Each dot represents one individual.

### Examples

### Example 1: Function of Orai1 in Platelet SOCE and Activation

Using reverse transcriptase (RT)-PCR analysis, we found Orai1 to be the predominant member of the Orai family present in human platelets at mRNA level; however, very faint bands of Orai2 and Orai3 were also observed. Western-blot analysis of human platelet lysates demonstrated robust expression of Orai1, indicating that the channel might have a role in Ca²⁺ homeostasis in those cells (Fig. 1A).

To directly test the function of Orai1 in platelet SOCE and activation, we generated Orai1-null (Orai1^{-/-}) mice through disruption of the Orai1 gene by insertion of a gene-trap cassette into intron 2 as recently independently reported by Vig and coworkers (18). Mice heterozygous for the Orail-null mutation developed normally, while ∼60 % of the Orai1^{-/-} mice died shortly after birth for unknown reason. Surviving Orai1^{-/-} animals developed significantly slower reaching only ∼60 % of the body weight of their littermates at 2 weeks of age (Fig. 1B, C) and showing still very high mortality as all animals died latest 4 weeks after birth. RT-PCR analysis revealed the presence of wild-type Orai1 mRNA message in control but not in Orai1^{-/-} platelets (Fig. 1 D). Western blot detection of Orai1 was not possible as no antibodies are available that recognize the murine protein. Similar to human platelets, low levels of Orai2 or Orai3 transcripts were detectable in both wild-type and Orai1^{-/-} platelets, whereas all three isoforms were strongly detectable in wild-type thymocytes (Takahashi (2007)) (Fig. 1 D). These results show that Orai1 is highly expressed in human platelets and suggest that Orai1 is also the dominant member of the Orai family in mouse platelets. Therefore, we analyzed Orai1^{-/-} platelets in more detail.

Due to the early lethality and growth retardation of Orai1^{-/-} mice, all further studies were performed with lethally irradiated wild-type mice transplanted with Orai1^{-/-} or control bone marrow cells. Four weeks after transplantation, both groups of mice had normal platelet counts (table 1) and RT-PCR confirmed the virtually complete absence of Orai1 mRNA in platelets from Orai1^{-/-} chimeras (Fig. 1 D). Furthermore, mean platelet volumes (MPV) and the expression of prominent surface glycoprotein receptors were similar between wild-type and Orai1^{-/-} chimeras (data not shown), as were the main hematological and clotting parameters (Table 1). Together, these results demonstrate that megakaryopoiesis and platelet formation occur independently of Orai1.

To test the role of Orai1 in SOCE, we performed intracellular calcium measurements in Orai1^{-/-} and control platelets. For this, Fura-2 loaded cells were treated with the sarcoplasmic/endoplasmic reticulum Ca²⁺ ATPase (SERCA) inhibitor thapsigargin (TG) in calcium free buffer followed by addition of extracellular calcium, and changes in [Ca²⁺]ᵢ were monitored (Fig. 1E left panel). Store release evoked by TG was comparable between wild-type and Orai1^{-/-} platelets (78.8 ± 25.7 nM and 62 ± 13.4 nM respectively, p=0.17, n=6) whereas it was reduced in Stim1^{-/-} platelets (42.3 ± 7 nM, p=0.005, n=6) as reported previously (Varga-Szabo (2008)). However, the subsequent SOCE was almost completely blocked in the absence of Orai1 (1438 ± 466 nM vs. 155 ± 44 nM, p<0.0001, n=6; Fig. 1E right panel) and this defect was similar to that seen in Stim1^{-/-} platelets (Fig. 1E, right panel and Ref. (Varga-Szabo (2008)). These results establish Orai1 as the principal SOC channel in platelets and show that its loss cannot be functionally compensated by Orai2 or Orai3. Furthermore, these data indicate that Orai1, in contrast to STIM1, is not required for proper store content regulation in platelets.

To investigate the impact of the Orai1-null mutation on agonist induced Ca²⁺ responses, we measured the changes in [Ca²⁺]ᵢ upon platelet activation with different agonists (Fig. 2A). In agreement with the results from the TG experiments, store release in response to ADP, thrombin (Fig. 2C) and the stable TxA2 analog U46619 (not shown) which act on Gq/PLCβ-coupled receptors was unaltered in Orai1^{-/-} platelets compared to wild-type. Furthermore, only a very mild reduction was seen in response to collagen-related peptide (CRP), a specific ligand of the activating collagen receptor glycoprotein VI (GPVI) that triggers tyrosine phosphorylation cascades downstream of the receptor-associated immunoreceptor tyrosine-based activation motif (ITAM) culminating in the activation of PLCγ2 (69.6 ± 15.9 nM vs. 50.5 ± 14.4 nM, p<0.05, n=6; Fig. 2A, B). These results again differ from those obtained with Stim1^{-/-} platelets where store release was strongly reduced in response to all these agonists further indicating a direct role for STIM1 in store content regulation. When the experiment was performed in the presence of extracellular calcium, however, a pronounced Ca²⁺ influx was detectable in wild-type platelets which was dramatically reduced, but not abrogated in Orai1^{-/-} platelets (Fig. 2A, B) and thereby similarly defective as previously seen in Stim1^{-/-} platelets (Varga-Szabo (2008)). Together, these results demonstrated that Orai1 is essential for efficient agonist induced Ca²⁺ entry in platelets but that it is not required for store content regulation in those cells. As a consequence, due to normal store release, Orai1^{-/-} platelets reach significantly higher cytosolic Ca²⁺ concentrations in response to all major agonists than Stim1^{-/-} platelets despite equally defective SOCE.

To test the functional consequences of the defective SOCE, we first performed in vitro aggregation studies. All agonists induced a comparable activation-dependent change from discoid to spherical shape in control and Orai1^{-/-} platelets, which can be seen in aggregometry as a short decrease in light transmission following the addition of agonists. However, Orai1^{-/-} platelets aggregated normally in response to the G-protein coupled agonists ADP, thrombin (Fig. 2C) and U46619 (not shown), the responses to collagen, CRP (Fig. 2C) and the strong GPVI-specific agonist convulxin (CVX, not shown) were diminished at low agonist concentrations, whereas the defect was overcome at intermediate or high agonist concentrations. This selective impairment in GPVI-ITAM-mediated activation was confirmed by flow cytometric analysis of integrin αIIbβ3 activation and degranulation-dependent P-selectin surface exposure. As shown in Fig. 2D, Orai1^{-/-} platelets displayed markedly reduced responses to CRP or CVX (p<0.0001), even at high concentrations, whereas the responses to ADP and thrombin were not affected. As expected, the weak agonist ADP failed to induce P-selectin surface expression in wild-type and Orai1^{-/-} platelets. These results demonstrate that loss of Orai1-mediated SOCE specifically impairs GPVI-induced integrin activation and degranulation whereas G-protein coupled agonists, despite defective [Ca²⁺]ᵢ signaling, are still able to induce unaltered cellular activation in these assays. Similar observations have been made with Stim1^{-/-} platelets (Varga-Szabo (2008)).

Under physiological conditions platelet adhesion and aggregation occur in the flowing blood where high shear forces strongly influence these platelet functions. To test the significance of Orai1-mediated SOCE in thrombus formation under flow, we studied platelet adhesion to collagen in a whole blood perfusion assay at high arterial shear rates (1,700 s⁻¹). Wild-type platelets rapidly adhered to collagen and consistently formed stable three-dimensional thrombi which covered 43.6 ± 6.1 % of the total surface area at the end of the 4 min runtime (Fig. 2E). In sharp contrast, platelets from Orai1^{-/-} mice could barely form three-dimensional thrombi and the overall surface coverage was reduced by ∼60 % compared to the control (17.6 ± 5.2, p<0.0001, n=5) (Fig. 2E). The defect in three-dimensional thrombus formation became even more evident when the relative thrombus volume was measured and found to be reduced by ∼95 % (33 x 10⁹ ± 5.8 x 10⁹ vs. 2.1 x 10⁹ ± 1.8 x 10⁹ integrated fluorescence intensity (IFI) /mm², p<0.0001, n=5) (Fig. 2E). These results show that Orai1-mediated SOCE is essential for the formation of stable three-dimensional thrombi under high shear flow conditions in vitro.

To assess the significance of Orai1-mediated SOCE for platelet function in vivo, wild-type and Orai1^{-/-} chimeras were intravenously injected with collagen/epinephrine (150 µg / kg ; 60 µg / kg), which causes lethal pulmonary thromboembolism (Nieswandt (2001)). While all but one wild-type chimeras died within 20 min after injection due to asphyxia, 6 out of 7 Orai1^{-/-} chimeras survived the challenge (Fig. 3A). This protection was based on reduced platelet activation as platelet counts 30 min after challenge (or shortly before death in the wild-type animals) were significantly higher in Orai1^{-/-} compared to wild-type chimeras (5.24 ± 0.8 in Orai1^{-/-} vs. 2.16 ± 0.9 in wild-type x 10⁵/µl, p<0.005, n=4) and the number of obstructed pulmonary vessels was ∼50% less in the mutant animals (11 ± 2 vs. 19 ± 3 per histological section, p<0.005, n=4) (Fig. 3B).

Next we assessed arterial thrombus formation in vivo in a model of arterial thrombosis where the abdominal aorta is mechanically injured and blood flow is monitored by an ultrasonic perivascular Doppler flow meter. In this model, thrombus formation is triggered predominantly by collagen and thus occurs in an ITAM/PLCγ2-dependent manner (Gruner (2005)). Whereas all wild-type vessels occluded, blood flow stopped only in 6 of 10 Orai1^{-/-} chimeras. However, in 4 of these 6 vessels the thrombi embolized and consequently normal blood flow was found in 8 of 10 Orai1^{-/-} chimeras at the end of the 30 min observation period (Fig. 3C-F). In contrast, all vessels in wild-type chimeras occluded (Fig. 3C,D) and only 2 of 10 vessels embolized and remained open (Fig. 3D-F). Next, the mice were tested in a model of FeCl₃-induced injury of mesenteric arterioles where thrombus formation is largely driven by thrombin and less dependent on ITAM/PLCγ2 signaling (Renne (2005)). Interestingly, 14 of 15 Orai1^{-/-} chimeras were able to form occlusive thrombi in this model, and the process showed similar kinetics as compared to the wild-type controls (11/12 vessels occluded) (Fig. 3G, H). Together, these results demonstrate that Orai1-mediated SOCE is required for the stabilization of platelet-rich thrombi at sites of arterial injury under conditions where the process in mainly driven by GPIb-GPVI-ITAM-dependent mechanisms.

We have recently shown that STIM1 is an essential mediator in the pathogenesis of ischemic brain infarction indicating that SOCE in platelets is crucial for the stabilization of intravascular thrombi in this setting (Varga-Szabo (2008)). To directly test this hypothesis, we subjected Orai1^{-/-} chimeras to occlusion of the middle cerebral artery (MCAO) with a filament as described (Kleinschnitz (2007)). After one hour the filament was removed to allow reperfusion and the animals were followed for another 24h before the extent of infarctions was assessed quantitatively on 2,3,5-triphenyltetrazolium chloride (TTC)-stained brain slices. In Orai1^{-/-} chimeras, infarct volumes 24 hours after reperfusion were reduced to less than 30% of the infarct volumes in control chimeras (18.15 ± 12.82 mm³ vs. 64.54 ± 26.80 mm³, p<0.0001) (Fig. 4A). The Bederson score assessing global neurological function (1.69 ± 0.65 vs. 3.43 ± 1.13, p<0.01) and the grip test, which specifically measures motor function and coordination (4.5 ± 0.76 vs. 2.14 ± 1.21, p<0.01), revealed that Orai1^{-/-} chimeras developed less neurological deficits compared to controls (Fig. 4B, C). Serial magnetic resonance imaging (MRI) on living mice showed that ischemic infarcts on T2-w MRI in Orai1^{-/-} chimeras were markedly reduced compared to control chimeras 24 hrs after transient MCAO thus confirming our histological findings from TTC stained brain sections. This protective effect was sustained since no delayed infarct growth was observed between day 1 and day 5. Moreover, a highly sensitive MRI sequence for detection of blood was used to assess hemorrhagic transformation. In contrast to increased bleeding complications in this stroke model after GPIIb/IIIa blockade (Kleinschnitz (2007)), T2-weighted gradient echo images revealed no hypointensities indicative of intracranial hemorrhages after tMCAO in Orai1^{-/-} chimeras (Fig. 4D). This shows that neuroprotection did not occur in expense of bleeding complications despite altered platelet function. Routine histological assessment of infarcts on hematoxylin and eosin-stained paraffin sections confirmed the TTC- and MRI findings. In Orai1^{-/-} chimeras, infarcts were restricted to the basal ganglia while in control animals the neocortex was regularly involved (Fig. 4E). In accordance with the findings of the cerebral ischemia-reperfusion model we found only a minor bleeding tendency of the Orai1^{-/-} chimeras after amputating the tip of their tail (Fig. 4F).

### Example 2: Materials and Methods

Mice. Animal studies were approved by the Bezirksregierung of Unterfranken. Orai1^{-/-} mice were generated as described by Vig et al (18). Briefly, ES cell clone (XL922) was purchased from BayGenomics and microinjected into C57BI/6 blastocysts to generate Orai1 chimeric mice. After germ line transmission heterozygous and knockout animals were genotyped by Southern blot and PCR using mouse tail DNA. Homologous recombinant and wild type alleles were detected by external probe which is located in upstream region of exon1. External probe was amplified by PCR (ExtpFor: 5'-GCTAGGGGAATCTCAGAAAC-3'; ExtpRev:5'-CATCCGAGGTCACCTCTGGG-3'). For PCR based genotyping geo-specific forward and reverse primers were used (GeoF: 5'-TTATCGATGAGCGTGGTGGTTATG-3', GeoR: 5'-GCGCGTACATCGGGCAAATAATATC-3'). Generation of bone marrow chimeras. 5-6 weeks old C57BI/6 female mice were lethally irradiated with a single dose of 10 Gy, and bone marrow cells from wild type or Orai1^{-/-} mice were injected intravenously into the irradiated mice (4 x 10⁶ cells/mouse). All recipient animals received acidified water containing 2 g/I Neomycin sulphate for 6 weeks after transplantation.

RT-PCR analysis. Human and murine platelet mRNA was isolated using Trizol reagent and detected by reverse transcriptase (RT)-PCR, according to the manufacturer's protocol (Invitrogen). Primers were used as previously described (21).

Chemicals and antibodies. Anesthetic drugs: medetomidine (Pfizer, Karlsruhe, Germany), midazolam (Roche Pharma AG, Grenzach-Wyhlen, Germany), fentanyl (Janssen-Cilag GmbH, Neuss, Germany) and antagonists: atipamezol (Pfizer, Karlsruhe, Germany), flumazenil and naloxon (both from Delta Select GmbH, Dreieich, Germany) were used according to the regulation of the local authorities. ADP (Sigma, Deisenhofen, Germany), U46619 (Alexis Biochemicals, San Diego, USA), thrombin (Roche Diagnostics, Mannheim, Germany), collagen (Kollagenreagent Horm, Nycomed, Munich, Germany) and thapsigargin (Molecular Probes) were purchased. Monoclonal antibodies conjugated to fluorescein isothiocyanate (FITC) or phycoerythrin (PE), or DyLight-488 were from Emfret Analytics (Würzburg, Germany). Anti-Orai1 antibodies were from ProSci Incorporated (Poway, USA).

Intracellular calcium measurements. Platelets isolated from blood were washed, suspended in Tyrode's buffer without calcium, and loaded with fura-2/AM (5 µM) in the presence of Pluronic F-127 (0.2 µg/ml) (Molecular Probes) for 30 min at 37°C. After labeling, platelets were washed once and resuspended in Tyrode's buffer containing no or 1 mM Ca²⁺. Stirred platelets were activated with agonists, and fluorescence was measured with a PerkinElmer LS 55 fluorimeter. Excitation was alternated between 340 and 380 nm, and emission was measured at 509 nm. Each measurement was calibrated using Triton X-100 and EGTA.

Platelet aggregometry. Changes in light transmission of a suspension of washed platelets (200 µl with 0.5 x 10⁶ platelets/µl) were measured in the presence of 70 µg/ml human fibrinogen. Transmission was recorded on a Fibrintimer 4 channel aggregometer (APACT Laborgeräte und Analysensysteme, Hamburg, Germany) over ten minutes, and was expressed in arbitrary units with buffer representing 100% transmission.

Flow cytometry. Heparinized whole blood was diluted 1:20 with modified Tyrode-HEPES buffer (134 mM NaCl, 0.34 mM Na₂HPO₄, 2.9 mM KCI, 12 mM NaHCO₃, 20 mM HEPES [N-2-hydroxyethylpiperazine-N'-2-ethanesulfonic acid], pH 7.0) containing 5 mM glucose, 0.35% bovine serum albumin (BSA), and 1 mM CaCl₂. For glycoprotein expression and platelet count, blood samples were incubated with appropriate fluorophore-conjugated monoclonal antibodies for 15 min at RT and analyzed on a FACScalibur instrument (Becton Dickinson, Heidelberg, Germany). For activation studies, blood samples were washed twice with modified Tyrode-HEPES buffer, incubated with agonist for 15 minutes, stained with fluorophore-labeled antibodies for 15 minutes at RT, and then analyzed.

Adhesion under flow conditions. Rectangular coverslips (24 x 60 mm) were coated with 0.2 mg/ml fibrillar type I collagen (Nycomed, Munich, Germany) for 1 h at 37°C and blocked with 1% BSA. Heparinized whole blood was labeled with a Dylight-488 conjugated anti-GPIX lg derivative (0.2 µg/ml) and perfused through transparent flow chambers with a slit depth of 50 µm and equipped with collagen-coated (200 µg/ml) coverslips. Perfusion was carried out at RT using a pulse-free pump at high (1.700 s⁻¹) shear rates. During perfusion, microscopic phase-contrast images were recorded in real-time. The chambers were rinsed by a 10 min perfusion with Hepes buffer pH 7.45 at the same shear, and phase-contrast and fluorescent pictures were recorded from at least five different microscopic fields (40 x objectives). Image analysis was performed off-line using Metavue software (Visitron, Munich, Germany). Thrombus formation was expressed as the mean percentage of total area covered by thrombi, and as the mean integrated fluorescence intensity per mm².

Bleeding time. Mice were anesthetized and a 3 mm segment of the tail tip was removed with a scalpel. Tail bleeding was monitored by gently absorbing blood with filter paper at 20 second intervals, without making contact with the wound site. When no blood was observed on the paper, bleeding was determined to have ceased. Experiments were stopped after 20 minutes.

Aorta occlusion model. A longitudinal incision was used to open the abdominal cavity of anesthetized mice and expose the abdominal aorta. An ultrasonic flow probe was placed around the vessel and thrombosis was induced by a single firm compression with a forceps. Blood flow was monitored until complete occlusion occurred or 30 minutes had elapsed.

Pulmonary thromboembolism model. Anesthetized mice were injected with a mixture of 150 µg/kg body weight fibrillar collagen and 60 µg/kg body weight epinephrine. Mice were observed until death or 30 min long and the lungs were harvested and conserved in 4% paraformaldehyde.

Intravital microscopy of thrombus formation in FeCl₃ injured mesenteric arterioles. Four weeks after bone marrow transplantations, chimeras were anesthetized, and the mesentery was exteriorized through a midline abdominal incision. Arterioles (35-60µm diameter) were visualized with a Zeiss Axiovert 200 inverted microscope (x10) equipped with a 100-W HBO fluorescent lamp source, a HBO fluorescent lamp source, and a CooISNAP-EZ camera (Visitron, Munich Germany). Digital images were recorded and analyzed off-line using Metavue software. Injury was induced by topical application of a 3 mm² filter paper saturated with FeCl₃ (20%) for 10 sec. Adhesion and aggregation of fluorescently labeled platelets (Dylight-488 conjugated anti-GPIX lg derivative) in arterioles was monitored for 40 min or until complete occlusion occurred (blood flow stopped for > 1 min).

MCAO model. Experiments were conducted on 10-12 wk-old Orai1^{-/-} or control chimeras according to published recommendations for research in mechanism-driven basic stroke studies(29). Transient middle cerebral artery occlusion (tMCAO) was induced under inhalation anesthesia using the intraluminal filament (6021PK10; Doccol Company) technique (25). After 60 min, the filament was withdrawn to allow reperfusion. For measurements of ischemic brain volume, animals were sacrificed 24 h after induction of tMCAO and brain sections were stained with 2% 2,3,5-triphenyltetrazolium chloride (TTC; Sigma-Aldrich, Germany). Brain infarct volumes were calculated and corrected for edema as described. Neurological testing. Neurological function was assessed by two independent and blinded investigators 24 h after tMACO. Global neurological status was scored according to Bederson et al (30) and motor function was graded using the grip test as described (11;25).

Assessment of brain infarcts by MRI. MRI was performed 24 h and 5 d after stroke on a 1.5 T unit (Vision; Siemens) under inhalation anesthesia. A custom made dual channel surface coil was used for all measurements (A063HACG; Rapid Biomedical). The MR protocol included a coronal T2-w sequence (slice thickness 2 mm), and a coronal T2-w gradient echo CISS sequence (Constructed Interference in Steady State; slice thickness 1 mm). MR images were transferred to an external workstation (Leonardo; Siemens) for data processing. Visual analysis of infarct morphology and ICH was performed in a blinded manner.

Histology. Formalin-fixed samples embedded in paraffin (Histolab Products AB) were cut into 4-µm thick sections and mounted. After removal of paraffin, tissues were stained with hematoxylin and eosin (Sigma-Aldrich).

Statistics. Results from at least three experiments per group are presented as mean ± SD. Differences between wild-type and Orai1^{-/-} groups were assessed by 2-tailed Student's t test. MCAO model: Results are presented as mean ± SD. Infarct volumes and functional data were tested for Gaussian distribution with the D'Agostino and Pearson omnibus normality test and then analyzed using the two-tailed student's t-test. For statistical analysis, PrismGraph 4.0 software (GraphPad Software, USA) was used. P-values < 0.05 were considered statistically significant.

**Table 1.**

| | **Orai1^{+/+}** | **Orai1^{-/-}** |
|---|---|---|
| **platelets** | 8036 ± 215 | 8888 ± 153 |
| **MPV (fL)** | 5.27 ± 0.12 | 5.35 ± 0.19 |
| **Erythrocytes** | 9150 ± 198 | 8718 ± 291 |
| **HCT [%]** | 45.9 ± 0.99 | 42.6 ± 1.2 |
| **aPTT [sec]** | 38.7 ± 6.8 | 37.7 ± 2.9 |
| **QT[%]** | 9.4 ± 0.5 | 9.8 ± 0.7 |
| **INR** | 0.88 ± 0.08 | 0.83 ± 0.04 |

**Table 1. Hematology and hemostasis in Orai1^{-/-} chimeras.** Platelet and erythrocyte counts per nl and coagulation parameters for control and Orai1^{-/-} chimeras. The abbreviations are mean platelet volume (MPV), hematocrit (HCT), activated partial thromboplastin time (aPTT), quick test (QT), and international normalized ration (INR). Values given are mean values ± SD of 5 mice for each genotype.

### References

1. Ruggeri, Z.M., 2002, Platelets in atherothrombosis. Nat.Med. 8:1227-1234.
2. Nieswandt, B. and Watson S.P., 2003, Platelet-collagen interaction: is GPVI the central receptor? Blood 102:449-461.
3. Bhatt, D.L. and Topol E.J., 2003, Scientific and therapeutic advances in antiplatelet therapy. Nat.Rev.Drug Discov. 2:15-28.
4. Berridge, M.J. et al., 2003, Calcium signalling: dynamics, homeostasis and remodelling. Nat.Rev.MoI.Cell Biol. 4:517-529.
5. Feske, S, 2007, Calcium signalling in lymphocyte activation and disease. Nat.Rev.lmmunol. 7:690-702.
6. Baba, Y. et al., 2008, Essential function for the calcium sensor STIM1 in mast cell activation and anaphylactic responses. Nat.Immunol. 9:81-88.
7. Liou, J. et al. STIM is a Ca2+ sensor essential for Ca2+-store-depletion-triggered Ca2+ influx. Curr.Biol. 15:1235-1241.
8. Oh-Hora, M. et al., 2008, Dual functions for the endoplasmic reticulum calcium sensors STIM1 and STIM2 in T cell activation and tolerance. Nat.Immunol.
9. Zhang,S.L. et al., 2005, STIM1 is a Ca2+ sensor that activates CRAC channels and migrates from the Ca2+ store to the plasma membrane. Nature 437:902-905.
10. Grosse,J. et al., 2007, An EF hand mutation in Stim1 causes premature platelet activation and bleeding in mice. J.Clin.Invest 117:3540-3550.
11. Varga-Szabo, D. et al., 2008, The ER calcium sensor STIM1 is an essential mediator of arterial thrombosis and ischemic brain infarction. J. Exp. Med., in press
12. Rosado, J.A. et al., 2002, Endogenously expressed Trp1 is involved in store-mediated Ca2+ entry by conformational coupling in human platelets. J.Biol.Chem. 277:42157-42163.
13. Sage, S.O. et al., 2002, TRP channels and calcium entry in human platelets. Blood 100:4245-4246.
14. Lopez, J.J. et al., 2006, Interaction of STIM1 with endogenously expressed human canonical TRP1 upon depletion of intracellular Ca2+ stores. J.Biol.Chem. 281:28254-28264.
15. Varga-Szabo, D. et al., 2008, Store-operated Ca2+ entry in platelets occurs independently of transient receptor potential (TRP) C1. Pflugers Arch., in press.
16. Feske, S. et al., 2006, A mutation in Orai1 causes immune deficiency by abrogating CRAC channel function. Nature 441:179-185.
17. Vig, M., et al., 2006, CRACM1 is a plasma membrane protein essential for store-operated Ca2+ entry. Science 312:1220-1223.
18. Vig, M. et al., 2008, Defective mast cell effector functions in mice lacking the CRACM1 pore subunit of store-operated calcium release-activated calcium channels. Nat.lmmunol. 9:89-96.
19. Prakriya, M. et al., 2006, Orai1 is an essential pore subunit of the CRAC channel. Nature 443:230-233.
20. Yeromin, A.V. et al., 2006, Molecular identification of the CRAC channel by altered ion selectivity in a mutant of Orai. Nature 443:226-229.
21. Takahashi, Y. et al., 2007, Essential role of the N-terminus of murine Orai1 in store-operated Ca2+ entry. Biochem.Biophys.Res.Commun. 356:45-52.
22. Nieswandt, B. et al., 2001, Long-term antithrombotic protection by in vivo depletion of platelet glycoprotein VI in mice. J.Exp.Med. 193:459-469.
23. Gruner, S., et al., 2005, Relative antithrombotic effect of soluble GPVI dimer compared with anti-GPVI antibodies in mice. Blood 105:1492-1499.
24. Renne, T. et al., 2005, Defective thrombus formation in mice lacking coagulation factor XII. J.Exp.Med. 202:271-281.
25. Kleinschnitz, C. et al., 2007, Targeting platelets in acute experimental stroke: impact of glycoprotein Ib, VI, and IIb/IIIa blockade on infarct size, functional outcome, and intracranial bleeding. Circulation 115:2323-2330.
26. Huang, G.N., et al., 2006, STIM1 carboxyl-terminus activates native SOC, I(crac) and TRPC1 channels. Nat.Cell Biol. 8:1003-1010.
27. Yuan, J.P. et al., 2007, STIM1 heteromultimerizes TRPC channels to determine their function as store-operated channels. Nat.Cell Biol. 9:636-645.
28. Hechler, B. et al., 2003, A role of the fast ATP-gated P2X1 cation channel in thrombosis of small arteries in vivo. J.Exp.Med. 198:661-667.
29. Dirnagl, U., 2006, Bench to bedside: the quest for quality in experimental stroke research. J.Cereb.Blood Flow Metab 26:1465-1478.
30. Bederson, J.B., et al., 1986, Rat middle cerebral artery occlusion: evaluation of the model and development of a neurologic examination. Stroke 17:472-476.

## Claims

1. A pharmaceutical composition comprising an inhibitor of Orai1 or an inhibitor of an activator of Orai1 and optionally a pharmaceutically active carrier, excipient or diluent.

2. An inhibitor of Orai1 or an inhibitor of an activator of Orai1 for use in treating or preventing a disorder related to venous or arterial thrombus formation.

3. A method of treating or preventing a disorder related to venous or arterial thrombus formation comprising administering a pharmaceutically effective amount of an inhibitor of Orai1 or of an inhibitor of an activator of Orai1 to a subject in need thereof.

4. The pharmaceutical composition of claim 1 or the inhibitor of claim 2 or the method of claim 3 wherein the inhibitor is an antibody or fragments thereof, an aptamer, an siRNA, an shRNA, a miRNA, a ribozyme, an antisense nucleic acid molecule, modified versions of these inhibitors or a small molecule.

5. The pharmaceutical composition of any one of claims 1 or 4 further comprising in the same or a separate container an antagonist of G-protein coupled receptors or signaling pathways, such as P2Y1 inhibitors, P2Y12 inhibitors, aspirin and inhibitors of PAR receptors.

6. The inhibitor or pharmaceutical composition of any one of claim 1, 2, 4 or 5 wherein the disorder is related to venous or arterial thrombus formation is myocardial infarction, stroke, inflammation, complement activation, fibrinolysis, angiogenesis and/or diseases related to FXII-induced kinin formation such as hereditary angioedema, bacterial infection of the lung, trypanosome infection, hypotensitive shock, pancreatitis, chagas disease, thrombocytopenia or articular gout.

7. The inhibitor or pharmaceutical composition of claim 6 wherein said stroke is ischemic stroke.
